# EUROPÄISCHE PATENTANMELDUNG

(11) **EP 3 320 861 A1**
(43) Veröffentlichungstag der Anmeldung: **16.05.2018**
(21) Anmeldenummer: 16198899.3
(22) Anmeldetag: 15.11.2016
(51) Int. Cl.: A61B 17/12

(54) **EINRICHTUNG ZUM POSITIONIEREN UND FREISETZEN EINES VERSCHLUSSIMPLANTATS ZUM VERSCHLIESSEN DES LINKEN HERZOHRS**

(71) Anmelder: Biotronik AG, 8180 Bülach (CH)
(72) Erfinder: Degen, Nicolas, 8222 Beringen (CH); Ulmer, Jens, 8700 Küsnacht (CH)
(74) Vertreter: Galander, Marcus

(57) **Zusammenfassung**

Das Dokument offenbart eine Einrichtung (10) zum Verschließen des linken Herzohrs (HO), mit: einem Verschlussimplantat (200) zum Verschließen des linken Herzohrs (HO), wobei das Verschlussimplantat (200) ein selbstexpandierbares Stentgerüst (2b) aufweist, das aus einem komprimierten Zustand in einen expandierten Zustand selbsttätig expandierbar ist, und wobei das Verschlussimplantat (200) eine am Stentgerüst (2b) festgelegte, flexible Materiallage (2d) aufweist, wobei die Materiallage (2d) im expandierten Zustand des Stentgerüstes (2b) aufgespannt ist, und einem Katheter (K) mit einem Lumen (L) zur Aufnahme des Verschlussimplantats (200) im komprimierten Zustand des Stentgerüstes (2b), wobei das Verschlussimplantat (200) durch Herausschieben aus dem Lumen (L) freisetzbar und das Stentgerüst (2b) in den expandierten Zustand überführbar ist. Erfindungsgemäß ist vorgesehen, dass die Einrichtung (10) ein an der flexiblen Materiallage festgelegtes, flexibles und längs erstrecktes Zugelement (2c) aufweist, so dass das Verschlussimplantat (200) mittels des Zugelementes (2c) wieder in das Lumen (L) des Katheters (K) einziehbar und dabei das Stentgerüst (2b) in den komprimierten Zustand überführbar ist.

## Beschreibung

Die Erfindung betrifft eine Einrichtung zum Verschließen des linken Herzohrs eines Herzens eines Patienten.

Derartige Einrichtungen sind z. B. aus der US 2014/0135817, US 2003/0181942 und US 2013/0138138 bekannt.

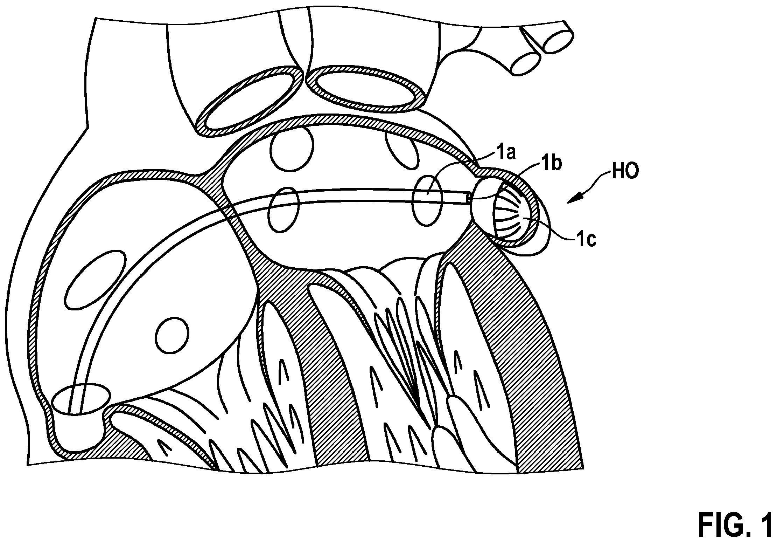

Solche selbstexpandierenden Systeme basieren in der Regel auf dem sogenannten pull-back-Prinzip, bei dem eine Katheteraußenhülle das Implantat in eine kollabierte Form zwingt und dieses durch Zurückziehen der Außenhülle wieder freisetzt. Aufgrund der Formgebung der Verschlussimplantate (meist tonnenförmig) kann die Freisetzung durch das pull-back-Prinzip weniger genau gesteuert werden, da es bei einem in etwa zu zwei Dritteln freigesetzten Verschlussimplantats zu einem sprunghaften Hervorschnellen des Verschlussimplantats kommen kann. Da es sich beim Herzohr um äußerst fragiles Gewebe handelt, ist ein hohes Verletzungsrisiko durch eine unkontrollierte Freisetzung eines Verschlussimplantats gegeben. Gemäß Fig. 1 sind weiterhin die heutigen Systeme bzw. Verschlussimplantate 1c durch eine Schraube 1b und insbesondere einen Führungsdraht fest mit dem Katheter 1a verbunden sind. Dies erschwert die Kontrolle einer erfolgreichen Platzierung dahingehend, dass die Fixierung des Implantats im Herzohrgewebe nicht hinreichend geprüft werden kann (z. B. kann ein fester Sitz des Implantats nach der Platzierung im Herzohr anhand eines Zugtests festgestellt werden).

Hiervon ausgehend besteht die Aufgabe der vorliegenden Erfindung darin, eine Einrichtung bereitzustellen, die eine kontrollierte und steuerbare Freisetzung des Verschlussimplantats ermöglicht.

Diese Aufgabe wird durch eine Einrichtung mit den Merkmalen des Anspruchs 1 gelöst. Vorteilhafte Ausführungsformen der Erfindung sind in den entsprechenden Unteransprüchen angegeben und werden nachfolgend beschrieben.

Gemäß Anspruch 1 wird eine Einrichtung zum Positionieren und Freisetzen eines Verschlussimplantats zum Verschließen des linken Herzohrs offenbart, mit: einem Verschlussimplantat zum besagten Verschließen des linken Herzohrs, wobei das Verschlussimplantat einen selbstexpandierbares Stentgerüst aufweist, das aus einem komprimierten Zustand in einen expandierten (z. B. entfalteten) Zustand selbsttätig expandierbar ist, und wobei das Verschlussimplantat eine am Stentgerüst festgelegte flexible Materiallage aufweist, wobei die Materiallage im expandierten Zustand des Stentgerüstes aufgespannt ist, und einem Katheter mit einem Lumen zur Aufnahme des Verschlussimplantats im komprimierten Zustand des Stentgerüstes, wobei das Verschlussimplantat durch Herausschieben aus dem Lumen freisetzbar und das Stentgerüst dabei in den expandierten Zustand überführbar ist.

Unter einer aufgespannten Materiallage im expandierten Zustand des Stentgerüstes wird im Rahmen dieser Anmeldung eine Materiallage verstanden, die die Querschnittsfläche des Stentgerüstes im Wesentlichen ausfüllt bzw. abdeckt. Aufgespannt ist hier also nicht im engen Sinne als "unter Spannung stehend" zu verstehen, sondern aufgespannt meint einen flächigen, im Wesentlichen entfalteten, Zustand der Materiallage.

Erfindungsgemäß ist vorgesehen, dass die Einrichtung ein an der flexiblen Materiallage festgelegtes, flexibles (insbesondere biegeschlaffes) und längs erstrecktes Zugelement aufweist, so dass das Verschlussimplantat mittels des Zugelementes wieder in das Lumen einziehbar und dabei das Stentgerüst in den komprimierten Zustand überführbar ist.

Die Fähigkeit zur Selbstexpansion des Stentgerüstes kann z. B. durch eine Materialeigenschaft des Stentgerüstes, wie z. B. eine elastische Rückstellkraft oder Superelastizität bedingt sein. Bevorzugt ist die Ausgestaltung des Stentgerüstes aus einer Formgedächtnislegierung, insbesondere Nitinol. Auch ein sonstiges mechanisches Wirkprinzip ist denkbar, um das selbsttätige Expandieren zu realisieren. Das Stentgerüst wird insbesondere durch seine Anordnung im Lumen des Katheters in seinem komprimierten Zustand gehalten und entfaltet sich, sobald es aus dem Lumen (durch eine Öffnung des Katheters hindurch) aus dem Lumen herausgeschoben wird (z. B. mittels eines im Lumen geführten Mittels, das zum Herausschieben des Verschlussimplantates geeignet ist).

Gemäß einer bevorzugten Ausführungsform der erfindungsgemäßen Einrichtung kann vorgesehen sein, dass das Zugelement ein Seil oder einen Faden aufweist. Insbesondere kann sich das Zugelement aus mehreren Strängen zusammensetzen, wobei ein solcher Strang zum Beispiel durch ein Seil oder einen Faden gebildet sein kann.

In einer bevorzugten Ausgestaltung der Erfindung ist das Zugelement als Kunststofffaden, bevorzugt aus Polyethylenterephtalat (PET), Polyamid (PA) und/oder Polypropylen (PP) ausgeführt, wobei der Faden sowohl aus einer einzelnen Faser (Strang) als auch aus mehreren einzelnen und miteinander verbundenen Fasern (Strängen) bestehen kann.

Weiterhin ist gemäß einer bevorzugten Ausführungsform der erfindungsgemäßen Einrichtung vorgesehen, dass die Materiallage aus einem Gewebe besteht, wobei das Zugelement durch das Gewebe der Materiallage geführt ist.

In dieser bevorzugten Ausgestaltung der Erfindung besteht die Materiallage aus einem Gewebe, welches derart ausgestaltet ist, dass das Zugelement durch das Gewebe geführt werden kann. In dieser Ausgestaltung der Erfindung wird die Materiallage derart ausgewählt, dass das Zugelement analog zu einem Faden beim Vernähen von Kleidung durch die Materiallage geführt werden kann.

Weiterhin ist gemäß einer bevorzugten Ausführungsform der erfindungsgemäßen Einrichtung vorgesehen, dass das Zugelement ein erstes und ein zweites Ende aufweist, wobei das Zugelement zwischen den beiden Enden eine Verdickung aufweist, so dass das Zugelement durch Ziehen am zweiten Ende vollständig aus der Materiallage herausziehbar ist, und so dass die Verdickung beim Ziehen am ersten Ende gegen die Materiallage stößt und das freigesetzte Verschlussimplantat über die Materiallage wieder in proximaler Richtung in das Lumen des Katheters einziehbar ist (hier zieht die Verdickung an der Materiallage/Gewebe und die Materiallage zieht am Stentgerüst).

Die proximale Richtung ist hier diejenige Richtung, in der das Verschlussimplantat wieder in den Katheter bzw. in das Lumen einziehbar ist, also vom Herzohr weg bewegbar ist. Entsprechend ist das proximale Ende des (im Katheter angeordneten) Stentgerüstes dasjenige Ende, das weiter vom Herzohr entfernt ist (das Stentgerüst wird mit seinem distalen Ende voran in das Herzohr eingeführt). Die zur proximalen Richtung entgegengesetzte Richtung ist die distale Richtung. Das Stentgerüst wird in der distalen Richtung aus dem Lumen des Katheters herausgeschoben und in das Herzohr eingeführt. Die oben beschriebene Verdickung des Zugelementes ist auf der distalen Seite der Materiallage angeordnet (entsprechend nimmt die Verdickung die Materiallage bzw. das Stentgerüst mit, wenn sie in die proximale Richtung gezogen wird).

Das Zugelement verläuft also bei einem im Lumen angeordneten Verschlussimplantat mit im komprimierten Zustand befindlichen Stentgerüst ausgehend vom ersten Ende im Lumen zur Materiallage hin, ist dort durch die Durchgangsöffnung der Materiallage geführt, wobei die Verdickung auf der distalen Seite der Materialage angeordnet ist (siehe oben), und erstreckt sich von dort an einem umlaufenden Randbereich der Materiallage vorbei zurück zum zweiten Ende.

Über die beiden Enden ist das Zugelement also handhabbar, wobei ggf. geeignete Betätigungsmittel der Einrichtung mit den Enden des Zugelementes gekoppelt sein können, so dass das Zugelement außerhalb des Körpers des Patienten mit den Betätigungsmitteln wie oben beschrieben ziehbar ist. Hierzu können die beiden Enden des Zugelements geeignet aus dem Lumen des Katheters herausgeführt sein.

Weiterhin ist gemäß einer bevorzugten Ausführungsform der erfindungsgemäßen Einrichtung vorgesehen, dass die Materiallage ein Gewebe ist. Bei der Materiallage kann es sich auch um ein sonstiges flexibles (insbesondere textiles) Flächengebilde handeln.

Insbesondere kann die Materiallage bzw. das Gewebe aus einem synthetischen Material bestehen, wie beispielsweise Polyethylenterephtalat (PET), Polyamid (PA), Teflon® und/oder Polypropylen (PP). Diese Materialien werden als Einzelfasern oder Faserbündel zu einem Gewebe verbunden. Ein derartiges Gewebe wird beispielsweise unter dem Handelsnamen Dacron® vertrieben.

In einer alternativen Ausgestaltung kann die Materiallage bzw. das Gewebe auch aus einem natürlichen Material wie Perikardgewebe oder bakterieller Zellulose bestehen.

Weiterhin ist gemäß einer bevorzugten Ausführungsform der erfindungsgemäßen Einrichtung vorgesehen, dass das Stentgerüst eine Mehrzahl an Streben aufweist, wobei die Streben jeweils einen ersten Endabschnitt sowie einen gegenüberliegenden zweiten Endabschnitt aufweisen, und wobei die ersten Endabschnitte der Streben miteinander verbunden sind (insbesondere integral miteinander verbunden sind), und wobei die zweiten Enden jeweils mit einem umlaufenden Randbereich der Materiallage verbunden sind.

Die Streben erstrecken sich insbesondere ausgehend von einer gemeinsamen Basis (die miteinander verbundenen ersten Endabschnitte) radial nach außen sowie gleichzeitig in axialer Richtung des Stentgerüstes, so dass die Streben zum Herstellen des komprimierten Zustandes mit ihren zweiten Endabschnitten in radialer Richtung aufeinander zu bewegbar sind, wobei sie benachbart zur (gedachten) axialen Richtung bzw. Stentachse zu liegen kommen.

Auf diese Weise weisen die zweiten Endabschnitte der Streben im komprimierten Zustand des Stentgerüstes eine geringere Beabstandung zueinander auf als im entfalteten Zustand, in dem die zweiten Endabschnitte in radialer Richtung derart voneinander beabstandet sind, dass die daran festgelegte Materiallage aufgespannt ist, insbesondere straff aufgespannt ist.

Zum Festlegen der besagten Materiallage an dem Stentgerüst ist gemäß einer Ausführungsform der erfindungsgemäßen Einrichtung bevorzugt vorgesehen, dass die zweiten Endabschnitte jeweils zumindest eine Ausnehmung (z. B. Unterschnitt oder Öse) aufweisen, wobei der umlaufende Randbereich der Materiallage mit der jeweiligen mindestens einen Ausnehmung über je einen Faden verbunden ist (z. B. durch Vernähen oder Knoten).

Weiterhin ist gemäß einer Ausführungsform vorgesehen, dass der umlaufende Randbereich der Materiallage die zweiten Endabschnitte der Streben zumindest abschnittsweise überdeckt. Hierdurch wird sichergestellt, dass die Materiallage mit dem umlaufenden Randbereich am Rand bzw. Ostium des Herzohres anliegen kann und eine entsprechende Dichtwirkung entfaltet.

Weiterhin ist gemäß einer bevorzugten Ausführungsform der erfindungsgemäßen Einrichtung vorgesehen, dass die Streben an den zweiten Endabschnitten je einen Vorsprung zum Verankern des Stentgerüstes im Ostium des Herzohres aufweisen. Die Vorsprünge können hierzu jeweils spitz zulaufen. Vorzugsweise erstrecken sich die Vorsprünge (bezogen auf einen expandierten Zustand des Stentgerüstes) jeweils ausgehend vom zweiten Endabschnitt der jeweiligen Strebe in radialer Richtung des Stentgerüstes nach außen.

Weiterhin ist gemäß einer Ausführungsform der Erfindung vorgesehen, dass die Streben in ihrer jeweiligen Erstreckungsebene jeweils ausgehend vom jeweiligen ersten Endabschnitt eine Rechtskrümmung und sodann eine Linkskrümmung aufweisen. Hierdurch kann eine Kelchform des expandierten Stentgerüstes realisiert werden, die eine effektive Verkürzung des Stentgerüstes in axialer Richtung möglich macht. Das Stentgerüst kann im expandierten Zustand jedoch alternativ auch konisch zulaufen.

Weiterhin ist gemäß einer bevorzugten Ausführungsform vorgesehen, dass die Materiallage im aufgespannten Zustand (d. h. bei expandiertem Stentgerüst) eine kreisförmige Oberfläche des Verschlussimplantats zum Verschließen des Herzohrs bildet.

Weiterhin ist gemäß einem Aspekt der vorliegenden Erfindung eine weitere Einrichtung zum Positionieren und Freisetzen eines Verschlussimplantats zum Verschließen des linken Herzohrs vorgesehen, mit: einem Verschlussimplantat zum Verschließen des linken Herzohrs, wobei das Verschlussimplantat einen selbstexpandierbares Stentgerüst aufweist, das aus einem komprimierten Zustand in einen expandierten Zustand selbsttätig expandierbar ist, und wobei das Verschlussimplantat eine am Stentgerüst festgelegte flexible Materiallage aufweist, wobei die Materiallage im entfalteten Zustand aufgespannt ist; und einem Katheter zum Transportieren des Verschlussimplantats an den Implantationsort, wobei die Einrichtung mindestens ein flexibles (insbesondere biegeschlaffes) und längs erstrecktes Zugelement aufweist, wobei das Stentgerüst mittels des Zugelementes in dem komprimierten Zustand haltbar ist, und wobei das Zugelement lösbar ist, so dass das Stentgerüst entfaltbar ist.

Das jeweilige Zugelement kann als Seil oder Faden ausgebildet sein oder kann ein Seil oder einen Faden aufweisen. In einer weiteren Ausgestaltung können auch mehrere Zugelemente verwendet werden.

Bevorzugt ist bei dem weiteren Aspekt der vorliegenden Erfindung vorgesehen, dass sich das Stentgerüst in einer axialen Richtung bzw. entlang einer Stentachse erstreckt, wobei das Stentgerüst eine Mehrzahl an durchgängig entlang der axialen Richtung erstreckten Balken aufweist, die parallel zueinander verlaufen und jeweils mit den in Umfangsrichtung benachbarten Balken über Stenthalbzellen verbunden sind, wobei die Streben der Stenthalbzellen eine geringere Breite als die Balken aufweisen, und wobei sich die Balken zu ihren Enden hin verjüngen, so dass ein komprimierter Zustand des Stentgerüstes z. B. durch ein mittig am Stentgerüst umlaufendes Zugelement herstellbar ist (z. B. durch Verengen einer durch das Zugelemente gebildeten Schlaufe).

Das Stentgerüst kann auf einem Katheter angeordnet werden, wobei im Lumen des Katheters die Zugelemente verlaufen. Diese können durch entsprechende Durchgangsöffnungen aus dem Katheter/Lumen herausgeführt sein, wobei sie jeweils um einen Balken des Stentgerüstes herumgelegt sind, so dass die das Stentgerüst in seinem komprimierten Zustand halten können. Das Stentgerüst kann dann mit seinem distalen Ende voran in das Herzohr geschoben werden, wobei die Zugelemente anschließend gelockert werden, so dass das Stentgerüst im Herzohr expandiert wird und die am proximalen Ende des Stentgerüstes festgelegte Materiallage aufgespannt wird und dabei das Herzohr verschließt. Die Zugelemente können anschließend über das Lumen des Katheters vollständig entfernt werden.

Weitere Merkmale und Vorteile der Erfindung sollen bei der Figurenbeschreibung von Ausführungsbeispielen der Erfindung anhand der Figuren erläutert werden. Es zeigen:
- Fig. 1: ein Verschlussimplantat nach dem Stand der Technik;
- Fig. 2: ein erfindungsgemäßes Verschlussimplantat einer erfindungsgemäßen Einrichtung;
- Fig. 3: eine weitere Ansicht des in der Figur 2 gezeigten Verschlussimplantats;
- Fig. 4: eine weitere Ansicht des in den Figuren 2 und 3 gezeigten Verschlussimplantats;
- Fig. 5: eine weitere Ansicht des in der Figuren 2 bis 4 gezeigten Verschlussimplantats;
- Fig. 6: eine schematische Schnittansicht des in den Figuren 2 bis 5 gezeigten Verschluss-implantats;
- Fig. 7: eine Seitenansicht des in der Figur 6 gezeigten Verschlussimplantats;
- Fig. 8: eine Ansicht eines in einem Lumen eines Katheters angeordneten Verschlussimplantats;
- Fig. 9: eine Ansicht der Freisetzung des Verschlussimplantats im Herzohr;
- Fig. 10 und 11: das Wiedereinziehen des Verschlussimplantats in das Katheterlumen (so genanntes Recapturing);
- Fig. 12 und 13: das erneute Freisetzen des Verschlussimplantats im linken Herzohr;
- Fig. 14: eine schematische Seitenansicht einer weiteren Einrichtung zum Positionieren und Freisetzen eines Verschlussimplantats;
- Fig. 15: eine schematische Schnittansicht einer der Einrichtung gemäß Figur 14 mit Verschlussimplantat/Stentgerüst im komprimierten Zustand;
- Fig. 16: eine weitere schematische Schnittansicht (senkrecht zur axialen Richtung) der in den Figuren 14 und 15 gezeigten Einrichtung mit expandiertem Stentgerüst;
- Fig. 17: eine weitere schematische Schnittansicht der in den Figuren 14 bis 16 gezeigten Einrichtung mit expandiertem Stentgerüst;
- Fig. 18: ein mittels eines umlaufenden Zugelementes komprimiertes Stentgerüst nach Art der Figuren 14 bis 17; und
- Fig. 19: ein axialer Balken des in der Figur 18 gezeigten Stentgerüstes sowie Abschnitte von Stenthalbzellen, über die der Balken mit in der Umfangsrichtung des Stentgerüstes benachbarten Balken verbunden ist.

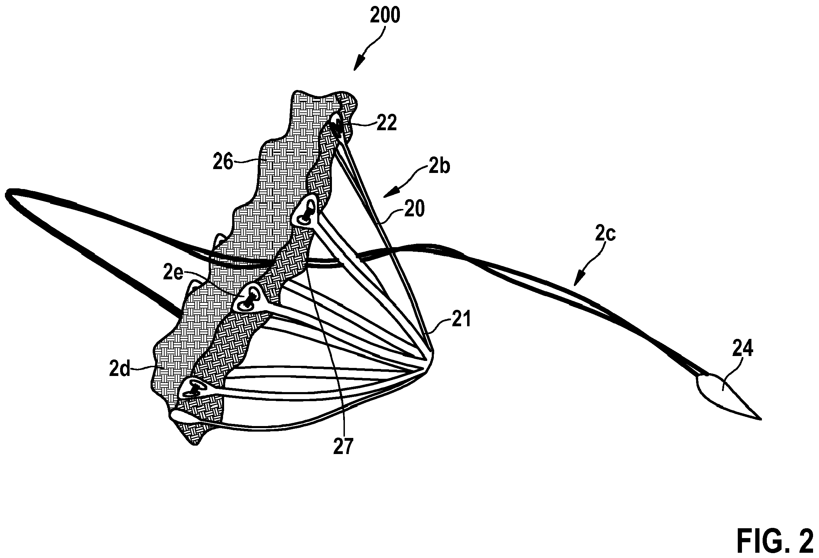

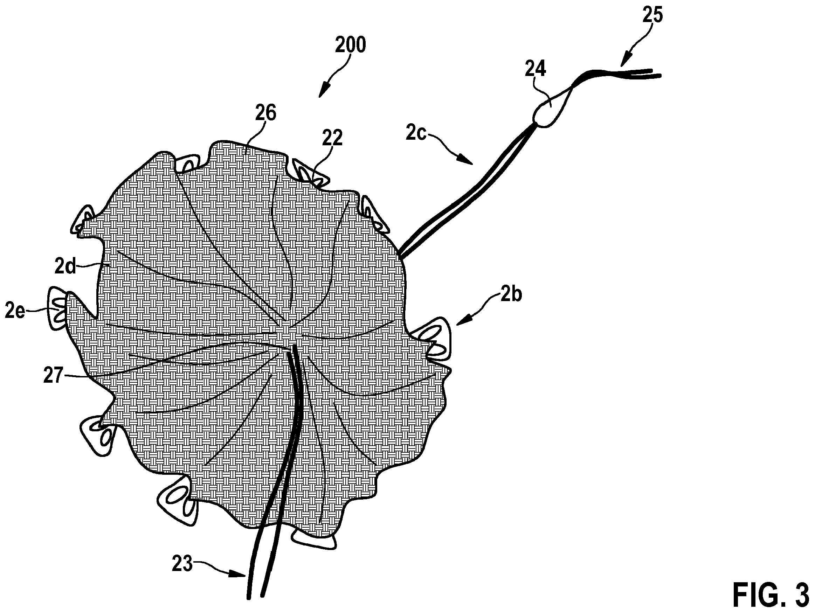

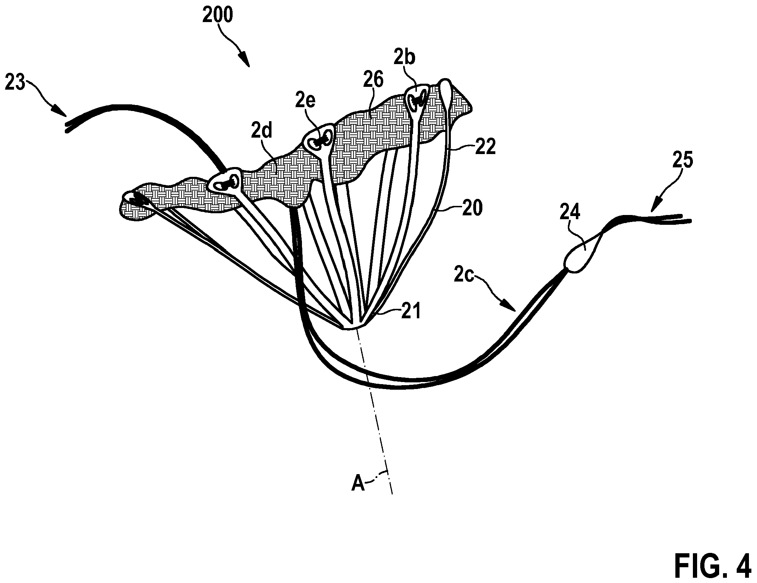

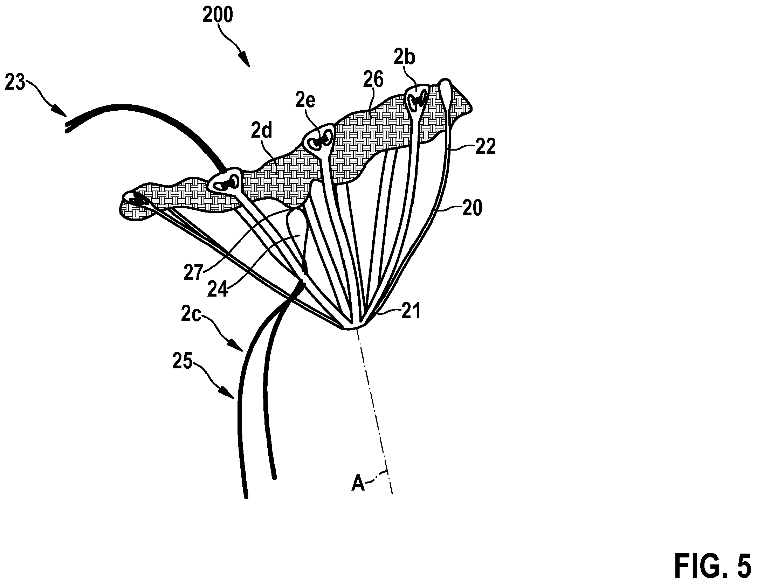

Die Figur 2 zeigt im Zusammenhang mit den Figuren 3 bis 13 eine erfindungsgemäße Einrichtung 10 zum Verschließen des linken Herzohrs HO eines insbesondere menschlichen Herzens, das z. B. in der Figur 1 schematisch dargestellt ist. Die Einrichtung 10 weist ein Verschlussimplantat 200 zum Verschließen des linken Herzohrs HO auf, wobei das Verschlussimplantat 200 ein selbstexpandierbares Stentgerüst 2b aufweist, das aus einem komprimierten Zustand in einen expandierten Zustand selbsttätig expandierbar ist, und wobei das Verschlussimplantat 200 eine am Stentgerüst 2b festgelegte, flexible Materiallage 2d aufweist, wobei die Materiallage 2d im expandierten Zustand des Stentgerüstes 2b im Wesentlichen aufgespannt ist. Das Stentgerüst 2b kann z. B. aus Nitinol oder einem anderen geeigneten Material gebildet sein.

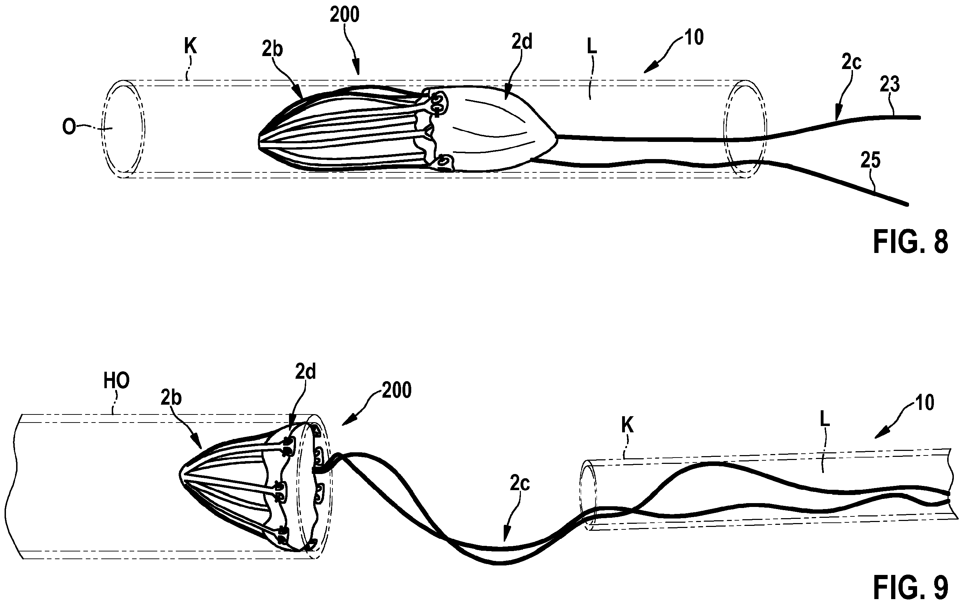

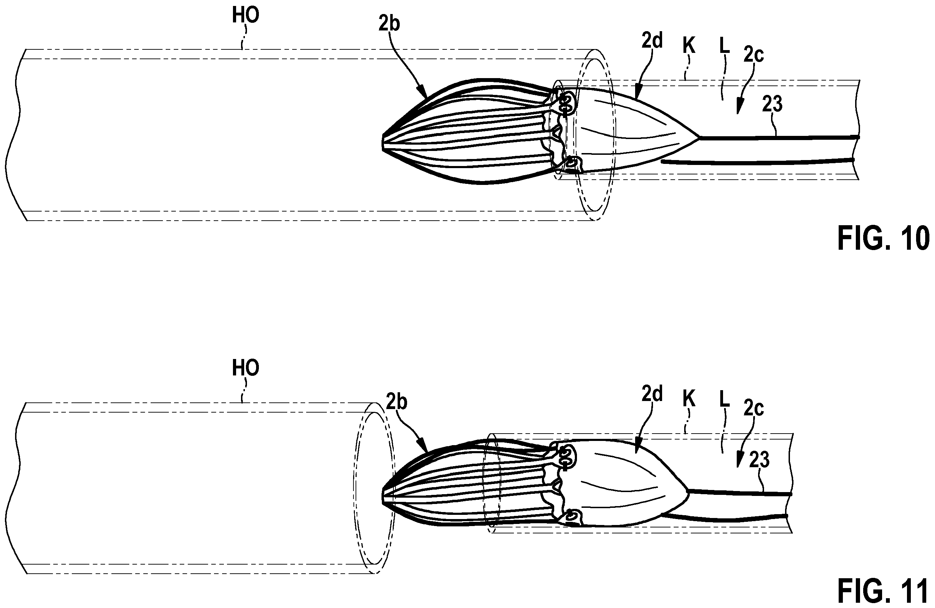

Weiterhin weist die Einrichtung 10 einen Katheter K mit einem Lumen L zur Aufnahme des Verschlussimplantats 200 im komprimierten Zustand des Stentgerüstes 2b auf, wobei das Verschlussimplantat 200 durch Herausschieben aus dem Lumen L (in die distale Richtung bzw. in der Figur 8 nach links) freisetzbar und das Stentgerüst 2b in den expandierten Zustand überführbar ist, der z. B. in den Figuren 2, 3, 4, 5, 6 und 7 gezeigt ist. Erfindungsgemäß ist vorgesehen, dass die Einrichtung 10 ein an der flexiblen Materiallage 2d festgelegtes, flexibles sowie insbesondere biegeschlaffes und längs erstrecktes Zugelement 2c aufweist, so dass das Verschlussimplantat 200 mittels des Zugelementes 2c wieder in das Lumen L des Katheters K einziehbar und dabei das Stentgerüst 2b in den komprimierten Zustand überführbar ist (vgl. Figuren 10 und 11).

Zum Herausschieben des Verschlussimplantats kann die Einrichtung 10 einen geeigneten Schieber (auch Pusher genannt) aufweisen, der im Lumen L des Katheters K angeordnet bzw. anordenbar ist und im Lumen L bewegbar ist (Schieber in den Figuren aus Gründen der Übersichtlichkeit nicht dargestellt). Bei dem Schieber kann es sich z. B. um ein Polymerlumen, eine Hypotube oder einen sogenannten Push-Wire handeln.

Wie z. B. in der Figur 3 angedeutet, kann das Zugelement 2c ein Seil oder einen Faden aufweisen, wobei das Zugelement 2c z. B. auch zwei oder mehrere Stränge aufweisen kann, bei denen es sich jeweils um ein Seil oder einen Faden handeln kann.

Wie des Weiteren aus der Fig. 3 ersichtlich ist, wird das Zugelement 2c an der Stelle 27 durch die Materiallage 2d geführt. Das Zugelement 2c passiert die Materiallage 2d dabei analog zu einem Faden, der bei dem Vernähen von Kleidung durch ein Gewebe geführt wird.

Das Zugelement 2c weist dabei ein erstes und ein zweites freies Ende 23, 25 auf, wobei das Zugelement 2c zwischen dem beiden Enden 23, 25 eine Verdickung 24 aufweist, d. h. einen Abschnitt mit erhöhtem Außendurchmesser, so dass das Zugelement 2c durch Ziehen am zweiten Ende 25 vollständig aus der Materiallage 2d herausziehbar ist, und so dass die Verdickung 24 beim Ziehen am ersten Ende 23 gegen die Materiallage 2c stößt, so dass bei einem freigesetzten Verschlussimplantat 200 das Verschlussimplantat 200 über die Materiallage wieder in proximaler Richtung in das Lumen L des Katheters K einziehbar ist. Die Materiallage 2d ist derart beschaffen, dass sie in sich hinreichend elastisch ist, um das Durchführen 27 des Zugelementes zu erlauben. Die innere Elastizität der Materiallage ist jedoch auch hinreichend klein, um zu verhindern, dass bei Kräften, mit denen üblicherweise bei der Handhabung des Zugelements 2c an diesem gezogen wird, die Verdickung 24 durch die Materiallage geführt werden kann. Bei der Materiallage 2d kann es sich insbesondere um ein Gewebe handeln, wo durch die Struktur des Gewebes ein Durchgang 27 für das Zugelement gebildet wird. Der im Gewebe bereits mikroskopisch vorhandene Platz zwischen den einzelnen, das Gewebe bildenden, Fasern erweitert sich elastisch zur Durchgangsöffnung 27 für das Zugelement 2c.

Bevorzugt ist die Verdickung 24 tropfenförmig ausgestaltet, wobei sich der Tropfen in Richtung des zweiten Endes 25 verjüngt. Dadurch kann das Zugelement nach Implantation des Verschlussimplantats 200 durch Zug in Richtung des zweiten Endes 25 leichter zwischen Herzohr und Verschlussimplantat aus dem Körper entfernt werden.

Über die beiden Enden 23, 25 ist das Zugelement 2c somit handhabbar, wobei ggf. geeignete Betätigungsmittel der Einrichtung mit den Enden 23, 25 gekoppelt sein können, die hier nicht näher ausgeführt werden.

Wie insbesondere in den Figuren 2 bis 7 zu erkennen ist, weist das Stentgerüst 2b eine Mehrzahl an Streben 20 auf, wobei die Streben 20 jeweils einen ersten Endabschnitt 21 sowie einen gegenüberliegenden zweiten Endabschnitt 22 aufweisen, wobei die ersten Endabschnitte 21 der Streben 20 miteinander verbunden sind, und wobei die zweiten Endabschnitte 22 jeweils mit einem umlaufenden Randbereich 26 der Materiallage 2c verbunden sind. Die Streben 20 laufen dabei - bezogen auf einen expandierten Zustand des Stentgerüstes 2b - ausgehend von einer gemeinsamen Basis bzw. den ersten Endabschnitten 21 in einer axialen Richtung A sowie jeweils in einer dazu senkrechten radialen Richtung, so dass die Streben 20 in Richtung auf den jeweiligen zweiten Endabschnitt 22 hin auseinander laufen. In diesem expandierten Zustand ist die Materiallage 2d, die an den zweiten Endabschnitten 22 festgelegt ist, aufgespannt. Im komprimierten Zustand des Stentgerüstes 2b hingegen weisen die zweiten Endabschnitte eine wesentlich geringere Beabstandung zueinander in radialer Richtung auf, so dass die Materiallage 2d entsprechend nicht aufgespannt ist.

Zum Festlegen des umlaufenden Randbereiches 26 der Materiallage 2d an den zweiten Endabschnitten 22 der Streben 20, weist der jeweilige Endabschnitt 22 insbesondere jeweils zumindest eine Ausnehmung 2e, insbesondere Öse, auf, wobei der umlaufende Randbereich 26 der Materiallage 2d mit der jeweiligen mindestens einen Ausnehmung 2e bzw. der entsprechenden Strebe 20 bevorzugt über je eine Naht bzw. einen Faden verbunden ist.

Die Materiallage 2d stellt sich also insbesondere alleine durch die radiale Expansion des Stentgerüstes 2b auf und ermöglicht somit den Abschluss des Herzohres HO zum Vorhof.

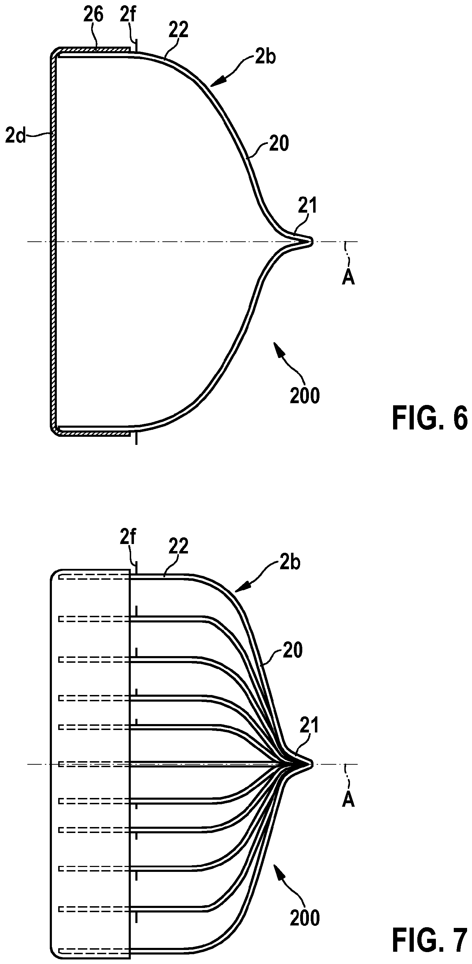

Weiterhin können die Streben 20 an den zweiten Endabschnitten 22 je einen Vorsprung 2f von maximal 1,5 mm zum Verankern des Stentgerüstes 2b im Ostium des Herzohres HO aufweisen (vgl. Figuren 6 und 7).

Gemäß einem Beispiel der Erfindung besteht die Materiallage 2d aus einem Gewebe, insbesondere aus einem textilen Gewebe aus Polyesterfäden (z.B. Dacron®), mit einer Maschenporosität, die insbesondere nicht größer ist als 0,15 mm.

Während das Zugelement 2c ausgehend vom ersten Ende 23 durch die Materiallage 2d geführt ist, ist das andere zweite Ende 25 seitlich an der Materiallage 2d bzw. dem Stentgerüst vorbeigeführt, so dass das Zugelement 2c hier auch seitlich an der Materiallage 2d in den Raum des Vorhofes vorbei geht.

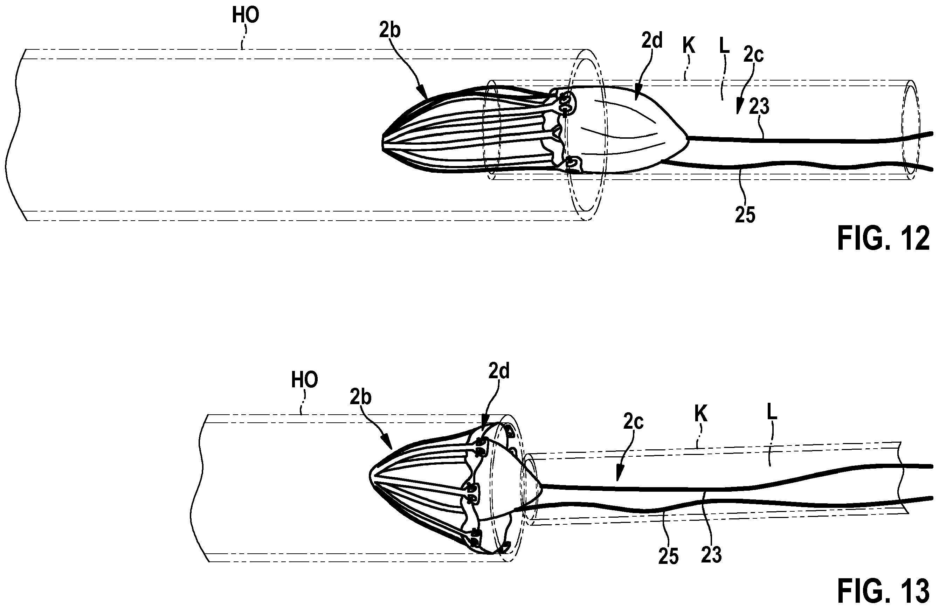

Nach der Implantation (vgl. Figuren 8 und 9) besteht nun die Möglichkeit, mittels des Zugelements 2c bzw. das Verschlussimplantat 200 durch Zug am ersten Ende 23 wieder in den Katheter K hereinzuziehen, wobei die Verdickung 24 die Materiallage 2d und somit das gesamte Verschlussimplantat 200 zieht (Figuren 10 und 11), und gegebenenfalls einen neuen Implantationsversuch zu starten (Figuren 12 und 13) oder die Prozedur abzubrechen. Nach der geglückten Implantation lässt sich durch Ziehen des seitlichen zweiten Endes 25 des Zugelementes 2c das gesamte Zugelement 2c mit der Verdickung 24 zwischen dem Ostium des Herzohres HO und der Materiallage 2d des Verschlussimplantats (auch Occluder genannt) herausziehen. Hierdurch wir eine Entkoppelung des Verschlussimplantats 200 vom Katheter K gewährleistet.

Weiterhin kann vorgesehen sein, dass die Streben 20 jeweils ausgehend vom jeweiligen ersten Endabschnitt 21 eine Rechtskrümmung und sodann eine Linkskrümmung aufweisen, wie es in den Figuren 6 und 7 angedeutet ist.

Hierdurch ergibt sich eine kelchartige Form einer gedachten Einhüllenden des expandierten Stentgerüstes 2b. Somit erhält man insbesondere einen größere zylindrischen Teil des Systems bzw. Stentgerüstes 2b und eine entsprechend größere Abdeckung zwischen dem Verschlussimplantat 200 und dem Herzohr HO. Weiterhin wird eine geringe Ausdehnung in das Herzohr OH realisiert (Reduktion der Punktierungsgefahr und flache Anatomien können besser abgebildet werden).

Das Zugelement 2c kann aus Nylon gebildet sein oder Nylon aufweisen (oder einen oder mehrere Fäden aus Nylon aufweisen). Andere vergleichbare biokompatible Fasern aus Multi- oder Monofilament sind ebenfalls denkbar. Die Verdickung 24 kann einen Körper aus Kunststoff aufweisen.

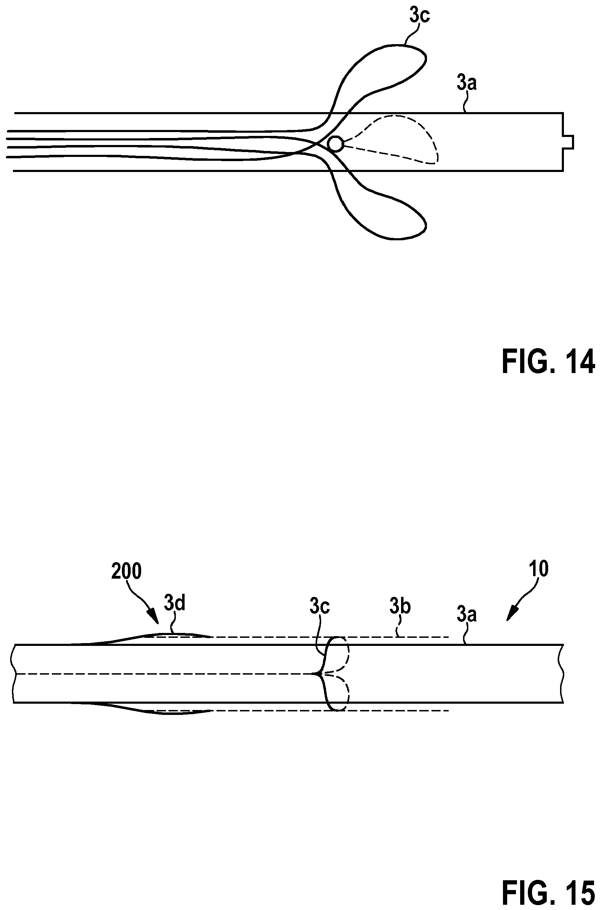

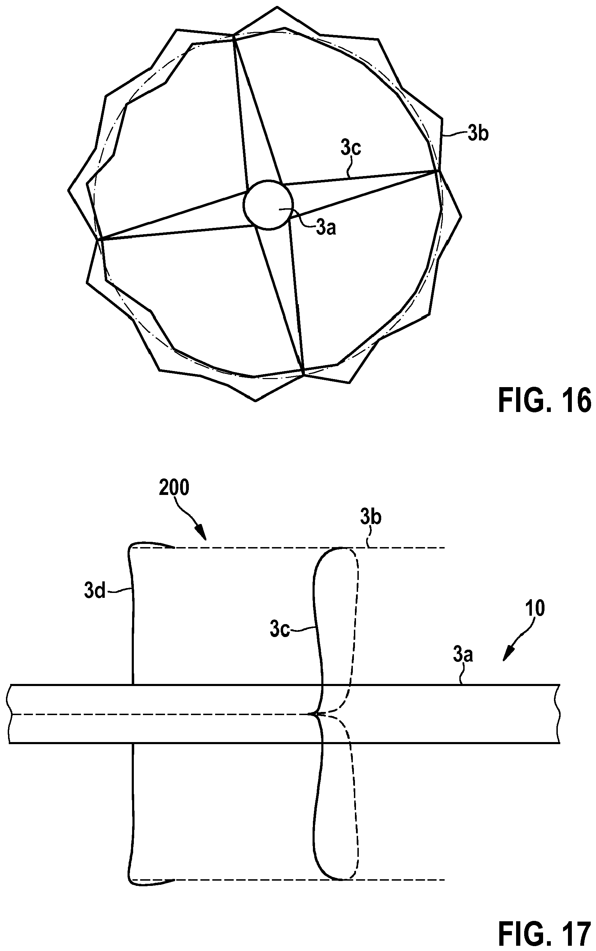

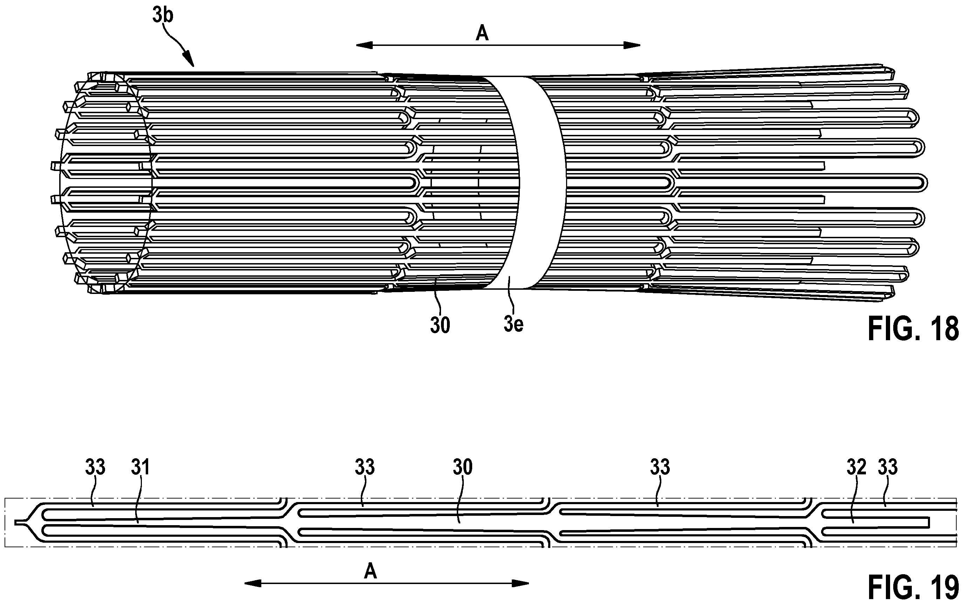

Weiterhin zeigen die Figuren 14 bis 17 eine weitere alternative Einrichtung 10 zum Positionieren und Freisetzen eines Verschlussimplantats 200 zum Verschließen des linken Herzohres HO, wobei auch hier die kontrollierte Freisetzung des Verschlussimplantats 200 nicht, wie sonst üblich, mit Hilfe eines pull-back-Systems erfolgt, sondern unter Zuhilfenahme von Zugelementen 3c bzw. eines Seilzugsystems 3c. Dazu wird bevorzugt ein zylindrisches Stentgerüst 3b mit Hilfe von zumindest einem Zugelement (z. B. Seil oder Faden) 3c kollabiert (vgl. z.B. Figur 18) und durch eine Schleuse eingeführt. Am Implantationsort wird der auf dem Katheter 3a sitzende Stent 3b durch Verstellen der Zugelemente 3c bzw. des Seilzugsystems 3c freigesetzt (vgl. Fig. 17) und bleibt mit diesen verbunden. Die Zugelemente 3c ermöglichen eine kontrollierte, schrittweise Freisetzung des Implantats 200, eine einfache Kontrolle der Fixierung des Verschlussimplantats 200 nach der Freisetzung und ggf. dessen Re-Positionierung. Zur der endgültigen Freisetzung wird das Seilzugsystem bzw. die Zugelemente 3c entfernt.

Insbesondere kann das Stentgerüst 3b mit Hilfe eines Seilzugsystems bestehend aus vier Zugelementen 3c gemäß Figuren 14 bis 17 auf einen Durchmesser von z. B. 12 Fr gecrimpt bzw. komprimiert werden. In diesem Zustand wird das Verschlussimplantat 200 mit Hilfe des Katheters 3a an den bestimmungsgemäßen Ort innerhalb des linken Herzohrs HO vorgeschoben und durch Relaxation der Zugelemente 3c (z.B. Fäden oder Seile) langsam und kontrolliert freigesetzt (vgl. Fig. 17). Um einen vollständigen Verschluss des Herzohres HO zu gewährleisten, ist eine Materiallage bzw. Abdeckung 3d vorgesehen, die auf der proximalen Seite des Stentgerüsts 3b z. B. durch Nähte fixiert wird. Die Materiallage 3d besteht z. B. aus einem textilen Gewebe (z. B. aus Polyesterfäden, insbesondere Dacron®) mit einer Maschenporosität, die insbesondere nicht größer ist als 0,1 mm.

Das Verschlussimplantat 200 bzw. das Stentgerüst 3b kann gemäß Fig. 18 grundsätzlich derart ausgestaltet sein, dass zum Crimpen bzw. Komprimieren des Stentgerüsts 3b nur ein mittig verlaufendes Zugelement (z.B. Faden oder Seil) 3e, das z. B. in Umfangrichtung mittig am Stent 3b umläuft, ausreicht, um das Gerüst 3b auf der gesamten Länge (entlang der axialen Richtung A) auf einen Durchmesser von z. B. 12 Fr zu verkleinern.

Dies wird gemäß Figur 19 durch die in der Stentachse bzw. in der axialen Richtung A längs verlaufenden durchgängigen Balken 30 des Stentgerüstes 3b erreicht. Die Balken 30 werden durch in der Umfangsrichtung zwischen den Balken 30 vorgesehene Stenthalbzellen 33 miteinander verbunden, wobei die Halbzellen 33 vorzugsweise eine um 50% geringere Breite als die Balken 30 aufweisen.

Weiterhin verjüngen sich die Balken 30 bevorzugt zu beiden Enden 31, 32 hin, so dass die größte Breite der Balken 30 in der Stentmitte vorliegt und insbesondere um 50% größer ist, als die Breite an den jeweiligen Balkenenden 31, 32. Dies ermöglicht es, dass die in der Mitte wirkende Kraft (durch das Seilzugsystem 3c bzw. die Zugelemente 3c auf die Balken übertragen, vgl. Fig. 16) groß genug ist, die beiden Balkenenden 31, 32 in gleicher Weise wie die Balkenmitte relativ zum Mittelpunkt der Längsachse A des Stents 3b zu verschieben.

## Patentansprüche

1. Einrichtung (10) zum Verschließen des linken Herzohrs (HO), mit
- einem Verschlussimplantat (200) zum Verschließen des linken Herzohrs (HO), wobei das Verschlussimplantat (200) ein selbstexpandierbares Stentgerüst (2b) aufweist, das aus einem komprimierten Zustand in einen expandierten Zustand selbsttätig expandierbar ist, und wobei das Verschlussimplantat (200) eine am Stentgerüst (2b) festgelegte, flexible Materiallage (2d) aufweist, wobei die Materiallage (2d) im expandierten Zustand des Stentgerüstes (2b) aufgespannt ist,
- einem Katheter (K) mit einem Lumen (L) zur Aufnahme des Verschlussimplantats (200) im komprimierten Zustand des Stentgerüstes (2b), wobei das Verschlussimplantat (200) durch Herausschieben aus dem Lumen (L) freisetzbar und das Stentgerüst (2b) in den expandierten Zustand überführbar ist,
**dadurch gekennzeichnet, dass**
die Einrichtung (10) ein an der flexiblen Materiallage (2d) festgelegtes, flexibles und längs erstrecktes Zugelement (2c) aufweist, so dass das Verschlussimplantat (200) mittels des Zugelementes (2c) wieder in das Lumen (L) des Katheters (K) einziehbar und dabei das Stentgerüst (2b) in den komprimierten Zustand überführbar ist.

2. Einrichtung nach Anspruch 1, **dadurch gekennzeichnet, dass** das Zugelement (2c) ein Seil oder einen Faden aufweist.

3. Einrichtung nach Anspruch 1 oder 2, **dadurch gekennzeichnet, dass** die Materiallage (2d) aus einem Gewebe besteht, wobei das Zugelement (2c) durch das Gewebe der Materiallage (2d) geführt ist (27).

4. Einrichtung nach einem der vorhergehenden Ansprüche, **dadurch gekennzeichnet, dass** das Zugelement (2c) ein erstes und ein zweites Ende (23, 25) aufweist, wobei das Zugelement (2c) zwischen dem beiden Enden (23, 25) eine Verdickung (24) aufweist, so dass das Zugelement (2c) durch Ziehen am zweiten Ende (25) vollständig aus der Materiallage (2d) herausziehbar ist, und so dass die Verdickung (24) beim Ziehen am ersten Ende (23) gegen die Materiallage (2c) stößt, so dass bei einem freigesetzten Verschlussimplantat (200) das Verschlussimplantat (200) wieder in das Lumen (L) des Katheters (K) einziehbar ist.

5. Einrichtung nach einem der vorhergehenden Ansprüche, **dadurch gekennzeichnet, dass** die Materiallage (2c) ein Gewebe ist.

6. Einrichtung nach einem der vorhergehenden Ansprüche, **dadurch gekennzeichnet, dass** das Stentgerüst (2b) eine Mehrzahl an Streben (20) aufweist, wobei die Streben (20) jeweils einen ersten Endabschnitt (21) sowie einen gegenüberliegenden zweiten Endabschnitt (22) aufweisen, wobei die ersten Endabschnitte (21) der Streben (20) miteinander verbunden sind, und wobei die zweiten Endabschnitte (22) jeweils mit einem umlaufenden Randbereich (26) der Materiallage (2c) verbunden sind.

7. Einrichtung nach Anspruch 6, **dadurch gekennzeichnet, dass** die zweiten Endabschnitte (22) im komprimierten Zustand des Stentgerüstes (2b) eine geringere Beabstandung zueinander aufweisen als im expandierten Zustand des Stentgerüstes (2b), in dem die zweiten Endabschnitte (22) derart voneinander beabstandet sind, dass die Materiallage (2d) aufgespannt ist.

8. Einrichtung nach Anspruch 6 oder 7, **dadurch gekennzeichnet, dass** die zweiten Endabschnitte (22) jeweils zumindest eine Ausnehmung (2e), insbesondere in Form einer Öse (2e), aufweisen, wobei der umlaufende Randbereich (26) der Materiallage (2d) mit der jeweiligen mindestens einen Ausnehmung (2e) über je eine Faden verbunden ist.

9. Einrichtung nach einem der Ansprüche 6 bis 8, **dadurch gekennzeichnet, dass** der umlaufende Randbereich (26) die zweiten Endabschnitte (22) der Streben (20) zumindest abschnittsweise überdeckt.

10. Einrichtung nach einem der Ansprüche 6 bis 9, **dadurch gekennzeichnet, dass** die Streben (20) an den zweiten Endabschnitten (22) je einen Vorsprung (2f), von bevorzugt maximal 1,5 mm Länge, zum Verankern des Stentgerüstes (2b) im Ostium des Herzohres (HO) aufweisen.

11. Einrichtung nach einem der Ansprüche 6 bis 10, **dadurch gekennzeichnet, dass** die Streben (20) jeweils ausgehend vom jeweiligen ersten Endabschnitt (21) eine Rechtskrümmung und sodann eine Linkskrümmung aufweisen.
